# EUROPÄISCHE PATENTSCHRIFT

(11) **EP 0 634 389 B1**
(45) Veröffentlichungstag und Bekanntmachung des Hinweises auf die Patenterteilung: **15.10.1997**
(21) Anmeldenummer: 94110395.4
(22) Anmeldetag: 04.07.1994
(51) Int. Cl.: C07C 68/00, C07C 69/96, B01J 12/00

(54) **Verfahren zur Herstellung von Dialkylcarbonaten**
Process for the preparation of dialkyle carbonates
Procédé de préparation de carbonates de dialkyle

(30) Priorität: 15.07.1993 DE 4323685
(43) Veröffentlichungstag der Anmeldung: 18.01.1995
(73) Patentinhaber: BAYER AG, 51368 Leverkusen (DE)
(72) Erfinder: Klausener, Alexander, Dr., D-50670 Köln (DE); Landscheidt, Heinz, Dr., D-47057 Duisburg (DE); Langer, Reinhard, Dr., D-47800 Krefeld (DE); Wagner, Paul, Dr., D-40597 Düsseldorf (DE)

(56) Entgegenhaltungen:
- EP-A- 0 523 728

## Beschreibung

Die vorliegende Erfindung betrifft ein Verfahren zur Herstellung von Dialkylcarbonaten durch Umsetzung von Kohlenmonoxid (CO) mit Alkylnitriten in Gegenwart eines heterogenen Platinmetall-Katalysators, wobei die Aktivität des Katalysators durch Zugabe geringer Mengen Halogen aufrechterhalten wird.

Dialkylcarbonate sind von allgemeiner chemischer und technischer Bedeutung. So ist beispielsweise Diethylcarbonat ein ausgezeichnetes Lösungsmittel im mittleren Siedebereich. Des weiteren sind die Dialkylcarbonate ausgezeichnete Carbonylierungs-und Acylierungsreagenzien. Sie haben große Bedeutung bei der Herstellung von anderen Carbonaten, von Urethanen und von Harnstoffen. Schließlich eignen sie sich aufgrund ihres hohen Sauerstoffgehalts als Treibstoffadditive zur Verbesserung der Klopffestigkeit von Ottokraftstoffen.

Es ist bekannt, Dialkylcarbonate durch Umsetzungen von Phosgen bzw. von Chlorameisensäurealkylestern mit Alkoholen herzustellen.

Es besteht jedoch ein steigendes Interesse daran, den Einsatz des giftigen Phosgens bzw. der davon abgeleiteten Zwischenprodukte wie Chlorameisensäureester durch andere Verfahren abzulösen.

Hier sind insbesondere Verfahren wichtig, in denen Kohlenmonoxid in der Gasphase mit Alkylnitrit an einem Platinmetall-Katalysator umgesetzt wird. Diesem Verfahren liegt die **Reaktionsgleichung (1)** zugrunde (Beispiel Dimethylcarbonat).

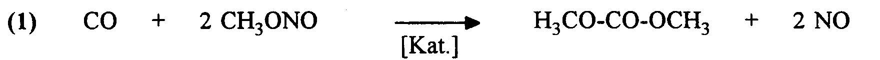

Methylnitrit selbst kann dazu auf an sich bekannte Weise in einer vorgeschalteten Reaktion gemäß einer der **Reaktionsgleichungen (2) - (5)** erzeugt werden.

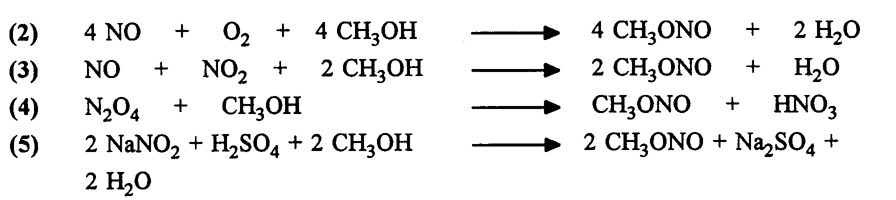

Die Herstellung von Dimethylcarbonat durch Umsetzung von Kohlenmonoxid und Methylnitrit in der Gasphase in Gegenwart eines heterogenen Katalysators, bei dem es sich bevorzugt um einen Platinmetall enthaltenden Trägerkatalysator, besonders bevorzugt um einen Palladium enthaltenden Trägerkatalysator und insbesondere bevorzugt um einen Palladiumhalogenid enthaltenden Trägerkatalysator handelt, ist verschiedentlich beschrieben worden, beispielsweise in folgenden wissenschaftlichen Publikationen oder Patentpublikationen:
JP 60/181 051; X.-Z. Jiang et al., Cuihua Xuebao **10(1)**, 75-78 (März 1989);
EP 425 197; X.-Z. Jiang, Platinum Metals Rev. **34(4)**, 178-180 (1990); EP 464 460; EP 503 091; EP 501 507; EP 503 618; EP 523 508; EP 523 728; EP 538 676.

Eine technische Ausführungsform dieses Prozesses wird in der Patentanmeldung EP 523 728 beschrieben. Diese Patentanmeldung beinhaltet die Rückführung der im Zuge der Umsetzung von Methylnitrit mit Kohlenmonoxid freigesetzten Stickoxide samt der nicht umgesetzten gasförmigen Reaktionspartner und des zur Inertisierung erforderlichen zusätzlichen Gases, bevorzugt Stickstoff, in einen dem eigentlichen Herstellungsprozeß des Dimethylcarbonats vorgeschalteten Verfahrensschritt, der der **Reaktionsgleichung (2)** entspricht und in dem durch Zuspeisung von Methanol und Sauerstoff und unter weitestgehender Entfernung des dabei freiwerdenden Wassers das für die Reaktion benötigte Methylnitrit regeneriert wird. Somit handelt es sich, bezogen auf die beteiligten gasförmigen Komponenten, namentlich bezüglich der Inertgase und Hilfsstoffe, der nicht umgesetzten gasförmigen Reaktanden, wie beispielsweise des nicht umgesetzten Methylnitrits und Kohlenmonoxids, sowie der beteiligten Stickoxide um einen Kreisprozeß.

Der eigentliche durch **Reaktionsgleichung (1)** beschriebene Herstellungsprozeß für beispielsweise Dimethylcarbonat läuft dabei an einem heterogenen Katalysator ab, der sich innerhalb eines Rohrbündelreaktors befindet. Als nachteilig dabei wirkt sich die hohe Temperaturbelastung des Katalysators aus, die aus der großen Reaktionswärme der gemäß **Reaktionsgleichung (1)** ablaufenden Umsetzung zwischen Kohlenmonoxid und Methylnitrit resultiert.

Das thermisch labile Methylnitrit wird unter diesen Bedingungen leicht zersetzt.

So ist es daher wenig überraschend, daß sich bei der in der genannten Patentanmeldung EP 523 728 beschriebenen Verfahrensweise 0,5 Gew.-% Formaldehyddimethylacetal, bezogen auf gebildetes Dimethylcarbonat, im Rohprodukt wiederfinden. Für manche Anwendungszwecke des Dimethylcarbonats können derartige Verunreinigungen aber nicht akzeptiert werden, wodurch umfangreiche Trenn-und Reinigungsschritte erforderlich werden.

Handelt es sich, wie beispielsweise bei dem genannten Formaldehyddimethylacetal, um leichtsiedende Stoffe, so kommen weitere Probleme durch das unweigerliche Akkumulieren derartig flüchtiger Komponenten innerhalb des gesamten zugrundeliegenden Kreisprozesses hinzu.Um die Anreicherung von Nebenprodukten innerhalb eines als technischen Kreisprozeß betriebenen Verfahrens zu verhindern, müssen festzulegende Anteile des zirkulierenden Kreisgases und der kondensierten Reaktionsprodukte, soweit es sich dabei nicht um Dimethylcarbonat selbst handelt, kontinuierlich oder diskontinuierlich, vorzugsweise kontinuierlich ausgeschleust werden. Hierdurch werden Rohstoffverluste verursacht und aufwendige Abgasbehandlungen erforderlich.

Es bestand daher die Aufgabe, die Umsetzung der gasförmigen Reaktanden Kohlenmonoxid und Alkylnitrit an einem heterogenen Katalysator so zu führen, daß die dabei freiwerdende Reaktionswärme schnell abgeführt und so das Auftreten von Zersetzungsprodukten des Methylnitrits minimiert wird.

Für Reaktionen mit extrem starker Wärmetönung und Katalysatoren bzw. Reaktionen mit extrem empfindlichem Temperaturverhalten ist eine kontinuierliche Thermostatisierung einzurichten.

Hierbei kann man den Katalysator beispielsweise entweder zwischen den Rohren oder auch innerhalb der Rohre eines Wärmetauschers unterbringen. (Linde-Reaktor gemäß der Patentanmeldung DE-OS 34 14 714, beziehungsweise Rohrbündelreaktor gemäß Chemie-Ingenieur-Technik **51** (1979), S. 257-265).

Reaktoren dieser Art mit Rohrdurchmessern von einem bis zu mehreren cm und Rohrlängen von 2-20 m sind seit langem Stand der Technik.

Trotz des ständigen Wärmeflusses in radialer Richtung kann sich, insbesondere in größeren Reaktoren, beispielsweise bei der Dimethylcarbonatsynthese aus Methylnitrit ein "hot-spot" innerhalb der Reaktorrohre ausbilden, der für Selektivitätsverluste infolge unerwünschter Bildung von Formaldehyddimethylacetal verantwortlich ist.

Überraschenderweise wurde gefunden, daß die Synthese von Dialkylcarbonat durch kontinuierliche Umsetzung von Kohlenmonoxid mit Alkylnitrit an einem heterogenen, ein oder mehrere Platinmetalle enthaltenden, bevorzugt Palladium enthaltenden Katalysator in der Gasphase bei kontinuierlicher Temperaturlenkung ohne Bildung von Nebenprodukten dadurch erreicht werden kann, daß das Katalysatorbett in Form einer oder mehrerer regelmäßig geformter flächiger Schichten mit einer Dicke von 0,01 - 50 cm, ausgebildet ist, wobei die Oberfläche der Katalysatorschichten durch eine für Gase durchlässige Schicht abgeschlossen wird und wobei diese Oberfläche auf der Eduktanströmseite und/oder auf der Produktaustrittsseite in einem Abstand von 0,1 - 10 cm einer in gleicher Weise regelmäßig geformten Wandung, die den Raum für die umzusetzenden Stoffe vom Raum für den Wärmeträger trennt, gegenübersteht, und eine solche Führung der umzusetzenden Stoffe vorgenommen wird, daß diese das Katalysatorbett senkrecht zu den flächigen Katalysatorschichten durchströmen.

Die beigefügten Zeichnungen zeigen den prinzipiellen Aufbau und die prinzipielle Wirkungsweise der erfindungsgemäßen Reaktoren und einige mögliche technische Ausführungsvarianten.

In den beigefügten Zeichnungen bedeuten die Pfeile mit durchgezogenen Linien und der Bezugsziffer (1) den jeweiligen Stoffstrom von der Zuführung des Edukts (1a) (Ausgangsstoff/-stoffgemisch, gegebenenfalls gemeinsam mit einem Verdünnungs- oder Trägergas) zur Abführung des Produkts (1b) (Reaktionserzeugnis/Reaktionserzeugnisgemisch, gegebenenfalls mit Verdünnungs-/Trägergas). Die Pfeile mit gestrichelten Linien und der Bezugsziffer (2) bedeuten den Wärmestrom vom Katalysator zum Wärmeträger.

Die punktierten, schmalen Flächen (3) bedeuten das durch eine für Gase durchlässige Schicht abgeschlossene Katalysatorbett; die für Gase durchlässige Schicht ist in den Zeichnungen nicht besonders hervorgehoben. Die waagerecht gestrichelten Flächen (4) bedeuten den durch eine Wandung abgeschlossenen Raum für den Wärmeträger.

Der Wärmeträger gilt in einer dem Fachmann bekannten Weise als durch den erfindungsgemäßen Reaktor bewegt und als mit einer externen Kühlung für die Temperaturlenkung verbunden. Wo es in den Zeichnungen beispielhaft gezeigt werden soll, wird der Wärmeträgerstrom durch Pfeile mit Doppelstrich und den Ziffern (4a) für den Eintritt bzw. (4b) für den Austritt bezeichnet.

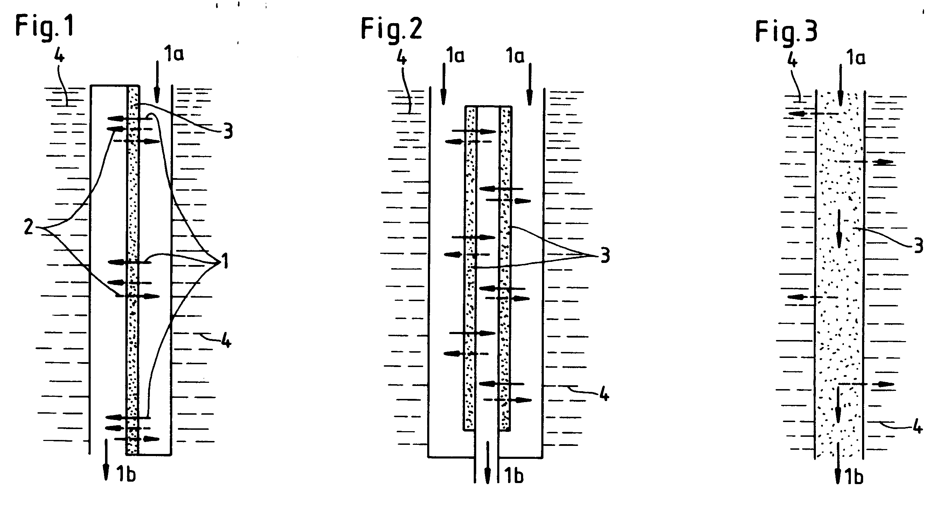

**Figur 1** zeigt das Prinzip des erfindungsgemäßen Reaktors am Schnitt durch ein ebenes, flaches Katalysatorbett mit gegenüberliegenden Wärmeträgerräumen. **Figur 2** zeigt das Prinzip am Schnitt durch ein als Hohlzylinder ausgebildetes Katalysatorbett. **Figur 3** zeigt im Gegensatz hierzu einen Rohrreaktor gemäß dem Stand der Technik, ebenfalls im Schnitt. Während beim erfindungsgemäßen Reaktor Stoff- und Wärmestrom stets parallel, antiparallel oder sowohl parallel als auch antiparallel zueinander verlaufen, stehen im herkömmlichen Reaktor Stoff-und Wärmestrom orthogonal zueinander. Folgerichtig sind im erfindungsgemäßen Reaktor die von Stoff- und Wärmestrom durchströmten Flächen stets identisch, während sie dies im herkömmlichen Reaktor nicht sein können.

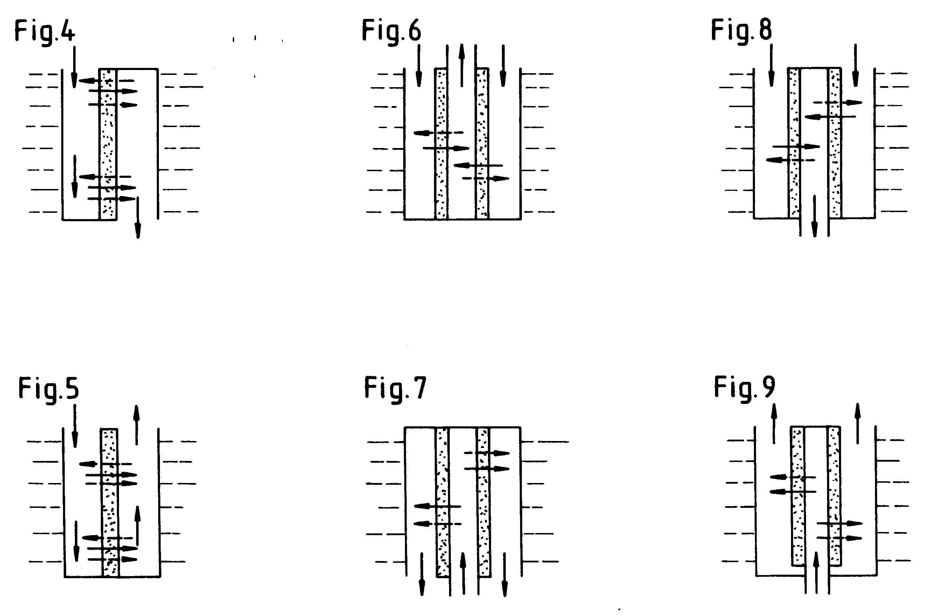

**Figuren 4 - 9** zeigen Variationen von **Figur 1** und **Figur 2**, bei denen in Abwandlung der Bauausführung - flaches Katalysatorbett oder Hohlzylinder als Katalysatorbett bzw. Zu- und Abfuhr von Edukt und Produkt - eine parallele oder antiparallele oder sowohl parallele als auch antiparallele Stoff- und Wärmefluß-Führung im Katalysatorbett gezeigt wird. Als Wärmefluß ist hierbei derjenige zu verstehen, der in der Richtung vom Katalysatorbett zur Wandung des Wärmeträgerraums strömt. Die zusätzliche Ziffernbezeichnung der Zeichnungssymbole wurde der Übersichlichtkeit halber in den **Figuren 4 - 9** fortgelassen. Es ist ersichtlich, daß die **Figuren 4** und **5** der **Figur 1** zugeordnet sind und die **Figuren 6 - 9** der **Figur 2**. In den **Figuren 4** und **5** bewegt sich der Wärmestrom sowohl parallel als auch antiparallel zum Stoffstrom, in den **Figuren 6 - 9** geschieht dies entweder parallel oder antiparallel.

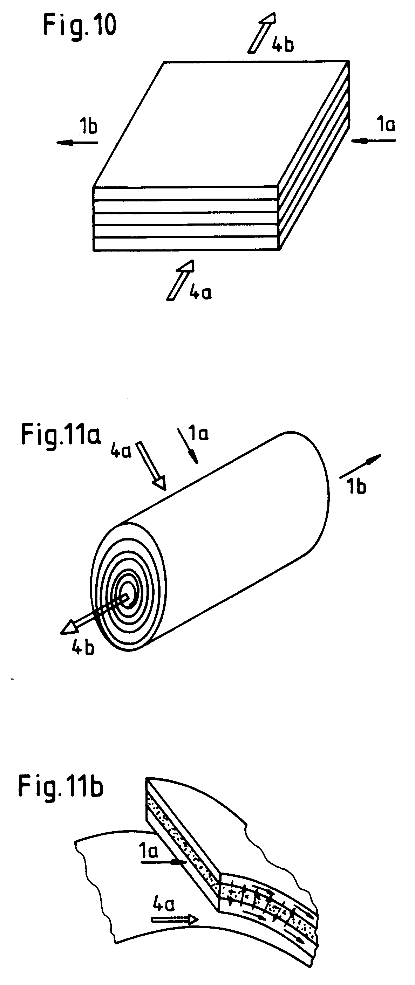

Das Katalysatorbett kann in einer oder mehreren flächigen Schichten ausgebildet werden. So können die flächigen Katalysatorschichten und die gegenüberstehenden, in gleicher Weise regelmäßig geformten Wandungen der Wärmeträgerräume in einer paketähnlichen Anordnung vereinigt werden (vgl. **Figur 10**). Die Kombination aus flächig angeordnetem Katalysator und Wärmeträgerraum kann jedoch auch in einer "aufgerollten" zylindrischen Form angeordnet werden (vgl. **Figur 11a**). Wenn hierbei in der Gesamtanordnung Stoff- und Wärmeträgerfluß auch in orthogonaler Richtung zueinander stehen, trifft dies in der oben geschilderten Weise für das Katalysatorbett im einzelnen nicht zu, wie in **Figur 11b** gezeigt wird.

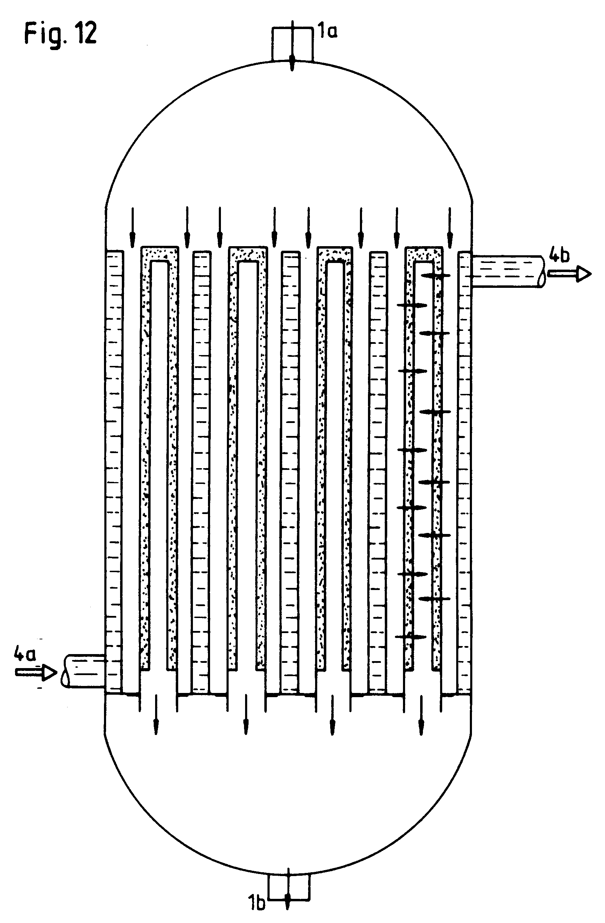

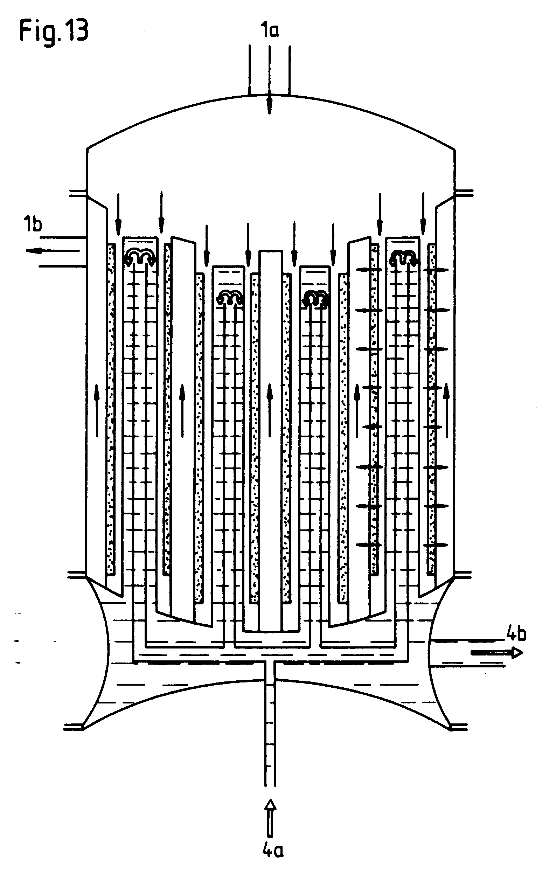

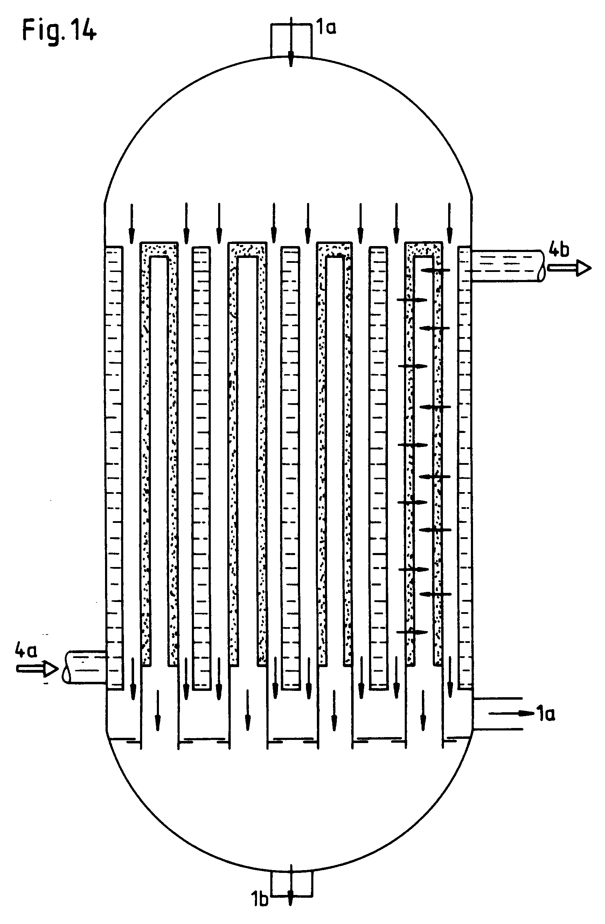

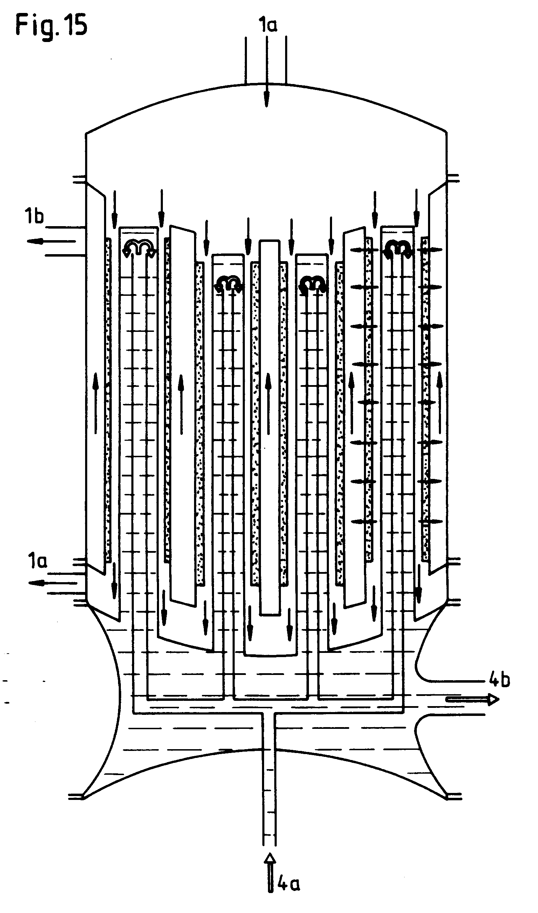

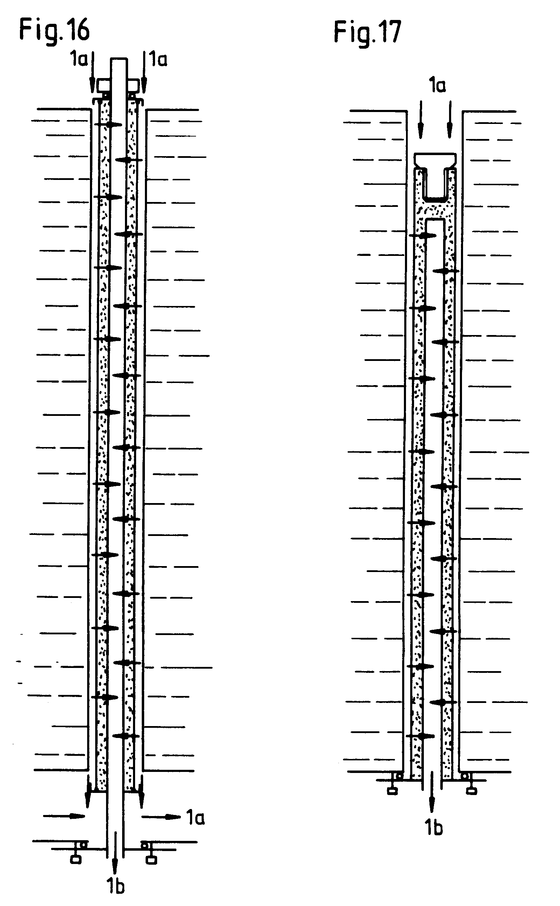

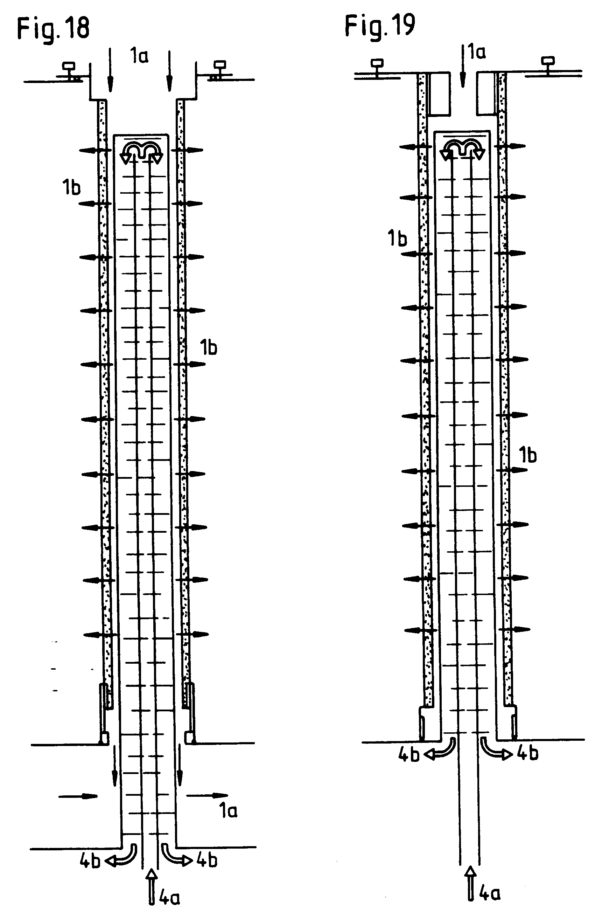

Die **Figuren 12 - 15** zeigen mögliche technische Ausführungen, bei denen Rohreinsätze in der Art herkömmlicher Rohrbündelreaktoren Verwendung finden. Die **Figuren 16 - 19** zeigen einzelne Rohreinsätze für solche Reaktoren, die eingeschraubt, eingeschweißt oder auf andere, grundsätzlich bekannte Weise in den Reaktorkörper eingesetzt werden können.

Die erfindungsgemäß einsetzbaren Reaktoren sind dadurch gekennzeichnet, daß das anströmende Edukt auf eine möglichst große Katalysatoroberfläche trifft und gleichzeitig eine möglichst große Fläche zum Wärmeaustausch realisiert wird. Das bedeutet, daß bei einer gegebenen Raum-Zeit-Belastung eine sehr niedrige Aufströmbelastung vorliegt und der Katalysator gleichmäßig temperiert ist. Das wiederum bedeutet, daß in den erfindungsgemäßen Reaktoren, anders als in den bekannten Reaktoren mit kontinuierlicher Thermostatisierung, der Wärmestrom bezüglich des Stoffstroms durch den Katalysator parallel, antiparallel oder parallel und antiparallel geführt wird. Im Zusammenhang hiermit besitzen die katalytisch aktiven Betten in den erfindungsgemäßen Reaktoren eine besonders geringe Ausdehnung in Strömungsrichtung. Diese bewegt sich bei 0,01 - 50 cm, bevorzugt bei 0,02 - 20 cm, besonders bevorzugt bei 0,05 - 10 cm, ganz besonders bevorzugt bei 0,2 - 2 cm. Die Katalysatorausschüttung wird in den erfindungsgemäßen Reaktoren durch poröse Wände begrenzt, die neben der Fixierung der Katalysatorschüttung die Aufgabe haben, die gleichmäßige Durchströmung des Katalysatorbetts über die gesamte Oberfläche zu garantieren. Hierzu muß der Strömungswiderstand dieser Wände und des Katalysatorbettes einen gewissen Mindestwert überschrieten, der vom Fachmann leicht experimentell oder durch Rechnung ermittelt werden kann. Im allgemeinen soll der Mindestströmungswiderstand der Katalysatorbetten einschließlich der porösen Gasverteilungswände Werte von 1 mbar bis 10 bar, bevorzugt von 2 mbar bis 1 bar, besonders bevorzugt von 5 mbar bis 500 mbar besitzen.

Der Abstand zwischen Katalysatorbett und Wandung beträgt 0,1 bis 10 cm, bevorzugt 0,2 bis 8 cm, besonders bevorzugt 0,3 bis 6 cm, ganz besonders bevorzugt 0,4 bis 4 cm.

Die porösen Wände in den erfindungsgemäßen Reaktoren bestehen bevorzugt aus Sinterwerkstoffen mit einer entsprechenden Porosität, wie sie zur Gasverteilung dem Fachmann bekannt sind. Besonders bevorzugt sind Sinterformkörper aus Metallen aufgrund ihrer guten Wärmeleitfähigkeit. Des weiteren sind auch Lochblendenkonstruktionen brauchbar. Auch sie müssen die Voraussetzungen erfüllen, den Eduktgasstrom ohne größere örtliche Schwankungen auf den Katalysator zu verteilen und eine gleichmäßige Durchströmung der Schüttung zu gewährleisten; hierzu ist ebenfalls wieder ein genügender Strömungswiderstand erforderlich, der auch mindestens teilweise von der Katalysatorschüttung erzeugt werden kann. Zu diesem Zweck muß das Katalysatormaterial genügend feinkörnig sein, um bei der geringen Durchdringungstiefe den erforderlichen Druckabfall in ausreichender Höhe zu gewährleisten. Es ist ferner verständlich, daß der Katalysator eine ausreichende Rieselfähigkeit haben muß, um beim Vorgang des Einfüllens eine einheitliche Packung zu gewährleisten; so darf das Katalysatormaterial beispielsweise nicht zur Verklebung neigen. Der Zusammenhang zwischen Partikelform und Partikelgröße einerseits und dem Strömungswiderstand einer Schüttung aus solchen Partikeln andererseits ist dem Fachmann bekannt und kann experimentell ermittelt werden.

Die regelmäßig geformten flächigen Schichten des Katalysatorbettes sind bevorzugt einseitig abgeschlossene Hohlzylinder mit Durchmessern von 2 bis 200 cm, bei Apparaten in technischem Maßstab bevorzugt 7 bis 100 cm, besonders bevorzugt 10 bis 50 cm; sie sind weiterhin bevorzugt in Form größerer Aggregate, z.B. als Rohrbündel, angeordnet.

Alkyl in Edukten und Produkten hat 1 bis 4 C-Atome und ist beispielsweise Methyl, Ethyl, Propyl, i-Propyl, Butyl oder i-Butyl, bevorzugt Methyl oder Ethyl, besonders bevorzugt Methyl.

Platinmetallkatalysatoren enthalten eines oder mehrere Metalle der Platinmetallgruppe wie Pd, Ru, Rh, Pt oder Ir, bevorzugt Pd oder Pd im Gemisch mit einem weiteren Platinmetall, besonders bevorzugt Pd allein. Das Platinmetall liegt als Salz vor, bevorzugt als Halogenid; es kann auch als Komplex vorliegen, z.B. als Li₂PdCl₄. Träger sind beispielsweise Kohle, Al₂O₃ (z.B. 103 \f "Symbol"-Al₂O₃), SiO₂, Alumosilikate, Zeolithe und andere dem Fachmann geläufige.

Das erfindungsgemäße Verfahren wird bei einem Druck von 0,1 bis 10 bar und einer Temperatur am Katalysator von 50 bis 150°C durchgeführt; die Temperatur wird kontinuierlich durch einen Wärmeträger gehalten bzw. gelenkt.

Das molare Verhältnis beträgt Alkylnitrit: CO = 0,1-10 : 1, bevorzugt 0,2 - 4 : 1, besonders bevorzugt 0,3 - 3 : 1. In bekannter Weise wird den Reaktionspartnern ein Inertgas und/oder das zugrundeliegende C₁-C₄-Alkanol zugesetzt.

### Beispiel

### a) Katalysatorherstellung

10 ml γ-Aluminiumoxid-Pellets mit einem Durchmesser von 2 - 3 mm wurden mit einer Lösung von Lithiumtetrachloropalladat in Wasser getränkt und bei 60-80°C im Vakuum getrocknet. Der Katalysator enthielt dann 8 g/l Palladium.

### b) Verfahrensbeschreibung

Der Reaktor bestand aus einem ölthermostatischen Glasrohr (Länge 62,0 cm, Innendurchmesser 4,9 cm). Konzentrisch im Innern des thermostatisierten Glasrohrs befand sich ein Rohr aus Glassinterwerkstoff. Dieses Rohr (Länge 15,0 cm, Durchmesser 2,0 cm, Porosität 3) war am oberen Ende zugeschmolzen und am unteren Ende an ein evakuiertes Doppelmantelglasrohr mit einem Kegelglasschliff (Normalschliff NS29) angeschmolzen (inneres Glasrohr mit Durchmesser 2,4 cm, äußeren Glasrohr mit Durchmesser 3,8 cm); der Raum zwischen innerem und äußerem Glasrohr war evakuiert. Durch das innere Glasrohr wurde das Produktgas abgeführt. Das äußere Glasrohr besaß, gemessen vom NS29-Kegelschliff bis zum unteren Ende des thermostatisierten Glasrohres, eine Länge von 25,0 cm. Am Ende des thermostatisierten Glasrohres schloß das äußere Glasrohr mit dem thermostatisierten Glasrohr gasdicht ab. Konzentrisch zum inneren Glassinterrohr befand sich ein weiteres Glassinterrohr im Inneren des ölthermostatisierten Glasrohrs (Länge 15,0 cm, Innendurchmesser 3,2 cm, Wandstärke 2 mm, Porosität 3). Dieses Glassinterrohr war am unteren Ende an eine NS29-Glasschliffhülse angeschmolzen, welche auf dem Glasschiffkern des Vakuumdoppelmantelrohres gasdicht aufsaß. Das äußere Glassinterrohr war am oberen Ende mit einem 10,0 cm langen Glasrohr mit NS29-Hülse verschmolzen. In dieser Hülse saß gasdicht ein ca. 10,0 cm langer evakuierter Glasschliffstopfen.

Zwischen dem äußeren und inneren Glassinterrohr befand sich die Katalysatorschüttung.

Der Ölmantel des Reaktors wurde auf 90°C thermostatisiert und ein Gasgemisch aus 55 Vol.-% Stickstoff; 20 Vol.-% Methylnitrit, 20 Vol.-% Kohlenmonoxid und 5 Vol.-% Methanol über den Katalysator geleitet. Dem Gasgemisch wurden 200 ppm HCl (Volumen) zugesetzt. Das dem Reaktor entströmende Gas wurde auf 5°C gekühlt, und die erhaltene kondensierte Phase wurde mittels Gaschromatographie untersucht.

Die nicht kondensierten Produkte wurden mittels IR-Spektroskopie und Massenspektroskopie erfaßt.

In dem das gebildete Dimethylcarbonat enthaltenden Reaktionsprodukt ließen sich weder Dimethyloxalat noch Ameisensäuremethylester und Formaldehyddimethylacetal analytisch nachweisen.

### c) Vergleichsbeispiel

In einem vertikal aufgestellten Rohrreaktor (Glas, Länge 50 cm, Durchmesser 4 cm) wurden zwischen eine Füllung aus Raschig-Ringen 10 ml des Katalysators aus **Beispiel a)** eingebracht.

Das Glasrohr wurde auf 90°C erhitzt, und ein Gasgemisch aus 55 Vol.-% Stickstoff, 20 Vol.-% Methylnitrit, 20 Vol.-% Kohlenmonoxid und 5 Vol.-% Methanol wurde hindurchgeleitet, wobei diesem Gasgemisch 200 ppm Chlorwasserstoffgas (Volumen) zugesetzt wurden. Das dem Reaktor entströmende Gas wurde auf 5°C gekühlt, und die erhaltene kondensierte Phase wurde mittels Gaschromatographie untersucht.

Die nicht kondensierten Produkte wurden mittels IR-Spektroskopie und Massenspektroskopie erfaßt.

Im erhaltenem Dimethylcarbonat wurden 0,03 Gew.-% Dimethyloxalat, 0,1 Gew.-% Ameisensäuremethylester sowie 0,04 Gew.-% Formaldehyddimethylacetal nachgewiesen.

## Patentansprüche

1. Verfahren zur Herstellung von Dialkylcarbonat durch kontinuierliche Umsetzung von Kohlenmonoxid mit Alkylnitrit in der Gasphase an einem heterogenen Katalysator, bevorzugt an einem heterogenen ein oder mehrere Platinmetalle enthaltenden Katalysator, besonders bevorzugt an einem heterogenen, Palladium enthaltenden Katalysator bei kontinuierlicher Temperaturlenkung mit Hilfe eines Warmeträgers, dadurch gekennzeichnet, daß das Katalysatorbett in Form von einer oder mehreren regelmäßig geformten flächigen Schichten mit eine Dicke von 0,01 - 50 cm ausgebildet ist, wobei die Oberfläche der Katalysatorschichten durch eine für Gase durchlässige Schicht abgeschlossen wird und wobei diese Oberfläche auf der Eduktanströmseite und/oder auf der Produktaustrittsseite in einem Abstand von 0,1 - 10 cm einer in gleicher Weise regelmäßig geformten Wandung, die den Raum für die umzusetzenden Stoffe vom Raum für den Wärmeträger trennt, gegenübersteht und eine solche Führung der umzusetzenden Stoffe vorgenommen wird, daß diese das Katalysatorbett senkrecht zu den flächigen Katalysatorschichten durchströmen.

2. Verfahren nach Anspruch 1, dadurch gekennzeichnet, daß das Katalysatorbett eine Dicke von 0,02 - 20 cm, bevorzugt 0,05 - 10 cm, besonders bevorzugt 0,2 - 2,0 cm hat.

3. Verfahren nach Anspruch 1, dadurch gekennzeichnet, daß der Abstand zwischen Katalysatorbett und Wandung 0,2 - 8,0 cm, bevorzugt 0,3 - 6,0 cm, besonders bevorzugt 0,4 - 4,0 cm beträgt.

4. Verfahren nach Anspruch 1, dadurch gekennzeichnet, daß die für Gase durchlässige Schicht zum Abschluß der Katalysatorschichten als Maschenwerk, Lochblende oder Sintermaterial, bevorzugt als Sintermaterial, besonders bevorzugt als Sintermetall ausgebildet ist.

5. Verfahren nach Anspruch 1, dadurch gekennzeichnet, daß das flächige Katalysatorbett einschließlich seiner für Gase durchlässigen abschließenden Schicht einen Mindestströmungswiderstand von 1 mbar bis 10 bar, bevorzugt von 2 mbar bis 1 bar, besonders bevorzugt von 5 - 500 mbar hat.

6. Verfahren nach Anspruch 1, dadurch gekennzeichnet, daß die regelmäßig geformten flächigen Schichten des Katalysatorbettes als einseitig abgeschlossene Hohlzylinder mit Durchmessern von 2 - 200 cm, bevorzugt 7 - 100 cm, besonders bevorzugt 10 - 50 cm ausgebildet sind, wobei in einer bevorzugten Ausführungsform mehrere von ihnen in Form größerer Aggregate, besonders bevorzugt als Rohrbündel angeordnet sind.

7. Verfahren nach Anspruch 1, dadurch gekennzeichnet, daß bei einem Druck von 0,1 - 10 bar gearbeitet wird.

8. Verfahren nach Anspruch 1, dadurch gekennzeichnet, daß die Katalysatortemperatur 50 - 150°C beträgt.

9. Verfahren nach Anspruch 1, dadurch gekennzeichnet, daß bei einem Volumenverhältnis von Alkylnitrit:Kohlenmonoxid = 0,1 bis 10:1 bevorzugt 0,2 bis 4:1, besonders bevorzugt 0,3 bis 3:1 gearbeitet wird.

10. Verfahren nach Anspruch 1, dadurch gekennzeichnet, daß als Katalysator ein Palladiumsalz, aufgebracht auf einen Träger, verwendet wird.

## Claims

1. Process for the preparation of dialkyl carbonate by continuous reaction of carbon monoxide with alkyl nitrite in the gas phase on a heterogeneous catalyst, preferably on a heterogeneous catalyst containing one or more platinum metals and particularly preferably on a heterogeneous catalyst containing palladium, with continuous temperature control using a heat transfer medium, characterized in that the catalyst bed takes the form of one or more flat layers of regular shape with a thickness of 0.01 - 50 cm, the surface of the catalyst layers being sealed by a gas-permeable layer, and this surface, on the starting-material feed side and/or on the product discharge side, facing a wall of similar regular shape, at a distance of 0.1 - 10 cm, which separates the space for the substances to be reacted from the space for the heat transfer medium, and the substances to be reacted being introduced in such a way as to flow through the catalyst bed perpendicularly to the flat catalyst layers.

2. Process according to Claim 1, characterized in that the catalyst bed has a thickness of 0.02 - 20 cm, preferably 0.05 - 10 cm and particularly preferably 0.2 - 2.0 cm.

3. Process according to Claim 1, characterized in that the distance between the catalyst bed and the wall is 0.2 - 8.0 cm, preferably 0.3 - 6.0 cm and particularly preferably 0.4 - 4.0 cm.

4. Process according to Claim 1, characterized in that the gas-permeable layer for sealing the catalyst layers takes the form of meshwork, a perforated screen or sintered material, preferably sintered material and particularly preferably sintered metal.

5. Process according to Claim 1, characterized in that the flat catalyst bed, including its gas-permeable sealing layer, has a minimum flow resistance of 1 mbar to 10 bar, preferably of 2 mbar to 1 bar and particularly preferably of 5 - 500 mbar.

6. Process according to Claim 1, characterized in that the regularly shaped, flat layers of the catalyst bed take the form of hollow cylinders, sealed on one side, with diameters of 2 - 200 cm, preferably 7 - 100 cm and particularly preferably 10 - 50 cm, several of them being arranged in the form of relatively large aggregates, particularly preferably as tube bundles, in a preferred embodiment.

7. Process according to Claim 1, characterized in that it is carried out at a pressure of 0.1 - 10 bar.

8. Process according to Claim 1, characterized in that the catalyst temperature is 50 - 150°C.

9. Process according to Claim 1, characterized in that it is carried out with an alkyl nitrite:carbon monoxide volume ratio of 0.1 to 10:1, preferably 0.2 to 4:1 and particularly preferably 0.3 to 3:1.

10. Process according to Claim 1, characterized in that a palladium salt applied to a support is used as the catalyst.

## Revendications

1. Procédé pour la préparation d'un carbonate de dialkyle par réaction continue du monoxyde de carbone avec un nitrite d'alkyle en phase gazeuse sur un catalyseur hétérogène, de préférence sur un catalyseur hétérogène contenant un ou plusieurs métaux de la série du platine, plus spécialement un catalyseur hétérogène contenant du palladium, avec contrôle continu de la température à l'aide d'un véhicule de chaleur, caractérisé en ce que le lit de catalyseur a la forme d'une ou plusieurs couches planiformes régulières d'une épaisseur de 0,01 à 50 cm, la surface des couches de catalyseur étant obturée par une couche perméable aux gaz, cette surface faisant face, du côté de l'arrivée des composants de départ et/ou du côté de la sortie des produits, à une distance de 0,1 à 10 cm, à une paroi de forme régulière analogue qui sépare l'espace réservé aux composants de réaction de l'espace réservé au véhicule de chaleur, et en ce que l'on conduit les composants de réaction en sorte qu'ils traversent le lit de catalyseur perpendiculairement aux couches planiformes de catalyseur.

2. Procédé selon la revendication 1, caractérisé en ce que le lit de catalyseur a une épaisseur de 0,02 à 20 cm, de préférence de 0,05 à 10 cm et plus spécialement de 0,2 à 2,0 cm.

3. Procédé selon la revendication 1, caractérisé en ce que la distance entre le lit de catalyseur et la paroi est de 0,2 à 8,0 cm, de préférence de 0,3 à 6,0 cm et plus spécialement de 0,4 à 4,0 cm.

4. Procédé selon la revendication 1, caractérisé en ce que la couche perméable aux gaz obturant les couches de catalyseur consiste en un matériau à mailles, une plaque perforée ou une matière frittée, de préférence une matière frittée et plus spécialement un métal fritté.

5. Procédé selon la revendication 1, caractérisé en ce que le lit planiforme de catalyseur, y compris la couche d'obturation perméable aux gaz, exerce une résistance minimale à l'écoulement de 1 mbar à 10 bar, de préférence de 2 mbar à 1 bar et dans les meilleures conditions de 5 à 500 mbar.

6. Procédé selon la revendication 1, caractérisé en ce que les couches planiformes de forme régulière du lit de catalyseur consistent en cylindres creux fermés d'un côté, à des diamètres de 2 à 200 cm, de préférence de 7 à 100 cm, et plus spécialement de 10 à 50 cm, et dans un mode de réalisation préféré, plusieurs de ces cylindres creux sont groupés en assemblages plus importants, de préférence sous la forme d'un faisceau tubulaire.

7. Procédé selon la revendication 1, caractérisé en ce que l'on opère sous une pression de 0,1 à 10 bar.

8. Procédé selon la revendication 1, caractérisé en ce que la température du catalyseur va de 50 à 150°C.

9. Procédé selon la revendication 1, caractérisé en ce que l'on opère à un rapport en volume nitrite d'alkyle/monoxyde de carbone de 0,1 à 10:1, de préférence de 0,2 à 4:1 et plus spécialement de 0,3 à 3:1.

10. Procédé selon la revendication 1, caractérisé en ce que l'on utilise en tant que catalyseur un sel de palladium appliqué sur un support.
